# EUROPEAN PATENT SPECIFICATION

(11) **EP 1 684 747 B1**
(45) Date of publication and mention of the grant of the patent: **09.07.2008**
(21) Application number: 04793891.5
(22) Date of filing: 08.10.2004
(51) Int. Cl.: A61K 31/404, A61P 35/00

(54) **BISINDOLYL MALEIMIDES USEFUL FOR TREATING PROSTATE CANCER AND AKT-MEDIATED DISEASES**
BISINDOLYLMALEIMID ZUR BEHANDLUNG VON PROSTATAKREBS UND AKT-VERMITTELTEN ERKRANKUNGEN
BISINDOLYLMALEIMIDES CONVENANT POUR LE TRAITEMENT DU CANCER DE LA PROSTAGE ET DE MALADIES INDUITES PAR AKT

(30) Priority: 24.10.2003 US 514291 P
(43) Date of publication of application: 02.08.2006
(73) Proprietor: ELI LILLY AND COMPANY, Indianapolis, Indiana 46285 (US)
(72) Inventor: GRAFF, Jeremy, Richard, Indianapolis, Indiana 46236 (US)
(74) Representative: Burnside, Ivan John
(86) International application number: PCT/US2004/030910
(87) International publication number: WO 2005/041953

(56) References cited:
- WO-A-01/30331
- WO-A-02/02116
- ROKHLIN, OSKAR W. ET AL: "Bisindolylmaleimide IX facilitates tumor necrosis factor receptor family-mediated cell death and acts as an inhibitor of transcription" JOURNAL OF BIOLOGICAL CHEMISTRY , 277(36), 33213-33219 CODEN: JBCHA3; ISSN: 0021-9258, 2002, XP009042295
- ZHONG H ET AL: "MODULATION OF HYPOXIA-INDUCIBLE FACTOR 1ALPHA EXPRESSION BY THE EPIDERMAL GROWTH FACTOR-PHOSPHATIDYLINOSITOL 3-KINASE/PTEN/AKT/FRAPPATHWAY IN HUMAN PROSTATE CANCER CELLS: IMPLICATIONS FOR TUMOR ANGIOGENESIS AND THERAPEUTICS" CANCER RESEARCH, AMERICAN ASSOCIATION FOR CANCER RESEARCH, BALTIMORE, MD, US, vol. 60, March 2000 (2000-03), pages 1541-1545, XP002931192 ISSN: 0008-5472
- TANAKA YUICHI ET AL: "Protein kinase C promotes apoptosis in LNCaP prostate cancer cells through activation of p38 MAPK and inhibition of the Akt survival pathway." JOURNAL OF BIOLOGICAL CHEMISTRY, vol. 278, no. 36, 5 September 2003 (2003-09-05), pages 33753-33762, XP001204595 ISSN: 0021-9258

## Description

### BACKGROUND OF THE INVENTION

Protein kinases are involved in the signal transduction pathways linking growth factors, hormones and other cell regulation molecules to cell growth, survival and metabolism under both normal and pathological conditions. One such protein kinase, protein kinase B (also known as AKT), is a serine/threonine kinase that plays a central role in promoting the proliferation and survival of a wide range of cell types, thereby protecting cells from apoptosis (programmed cell death) (Khwaja, Nature 33-34 (1990)). Three members of the AKT subfamily of second-messenger regulated serine/threonine protein kinases have been identified and are termed AKT-1, AKT-2, and AKT-3. A number of proteins involved in cell proliferation and survival have been described as substrates of AKT in cells. Two examples of such substrates include glycogen synthase kinase-3 (GSK3) and Forkhead transcription factors (FKs). *See* Brazil and Hemmings, Trends in Biochemical Sciences 26, 675-664.

A number of protein kinases and phosphatases regulate the activity of AKT. For instance, activation of AKT is mediated by phosphatidylinositol 3-kinase (PI3-K), which generates second messenger phospholipids that then bind to the pleckstrin homology (PH) binding domain of AKT. The binding attracts AKT to the plasma membrane where AKT is phosphorylated by phosphatidylinositol dependent kinase 1 (PDK1) at Thr308, which then triggers phosphorylation of AKT at Ser473 and activation of the enzyme. Amplifications of the catalytic subunit of PI3-K, p110α, or mutations in the PI3-K regulatory subunit, p85α lead to activation of AKT in several types of human cancer. (Vivanco and Sawyers, Nature Reviews in Cancer (2002) 2: 489-501).

The tumor suppressor, PTEN, is a critical negative regulator of AKT activation by PI3-K (Myers et al. Proc. Nat. Acad. Sci. 95, USA (1998) 13513-13518). Inactivating mutations in the *Pten* gene have been found at high frequencies in a large number of human tumors and tumor cell lines, including prostate cancer, breast cancer, ovarian cancer, glioblastoma, melanoma and other cancer types. Inactivation of the PTEN protein results in elevated levels of phosphorylated AKT and increased AKT activity in tumor cells (Li, et al., Science (1997) 275: 1943-1947; Guldberg, et al., Cancer Research (1997) 57: 3660-3663; Risinger, et al., Cancer Research (1997) 57: 4736-4738; Vivanco and Sawyers, Nature Reviews in Cancer (2002) 2: 489-501). In addition to overactivation of AKT due to defects in PTEN, direct amplification and/or overexpression of AKT-2 and AKT-3 have been found in human neoplasia, for example ovarian, pancreatic, prostate and breast cancer cells (Cheung et al., Proc. Nat. Acad. Sci. USA (1992) 89:9267-9271; Cheung et al., Proc. Nat. Acad. Sci. USA (1996) 93:3636-3641; Nakatani et al., J. Biol. Chem. (1999) 274:21528-21532).

The critical role of AKT in cell proliferation and survival is further strengthened by studies showing that germline knockout of AKT-1 results in partial embryonic lethality. The surviving littermates display stunted growth, increased organismal apoptosis, and early deaths. (Cho et al., J. Biol. Chem. (2001) 276: 38349-38520; Chen et al., Genes Dev. (2001) 15: 2203-2208). It has also been demonstrated that pharmacological inactivation of AKT induces apoptosis in cultured human ovarian cancer cells (Yuan et al., Oncogene 19, 2324-2340, 2000) and decreases growth of a human ovarian carcinoma xenograft in mice (Hu et al., Clin. Cancer Res. 6, 880-886, 2000).

Recent studies have also demonstrated the role of the PI3-K/AKT pathway in the life cycle of numerous viruses. Some viral proteins have been shown to directly activate the PI3-K/AKT pathway, thus providing an environment favorable for viral replication. These include the Tat protein of human immunodeficiency virus (HIV), Protein X of hepatitis B virus, and NS5A of hepatitis C virus (Borgatti et al., Eur. J. Immunol. (1997) 27: 2805-2811; Lee et al., J. Biol. Chem. (2001) 276: 16969-16977; He et al., J. Virol. (2002) 76: 9207-9217). The PI3-K/AKT pathway is also required for initiation and completion of the replication cycle of human cytomegalovirus (HCMV). In fact, pharmacological inactivation of this pathway results in abortive production of HCMV and survival of the host cells (Johnson et al., J. Virol. (2001) 75: 6022-6032).

Because of its pivotal role in the regulation of cell survival, the PI3 kinase/ AKT pathway provides a novel therapeutic target for the effective treatment of various disorders, particularly cancer and viral infections. However, such treatment requires the development of potent, selective inhibitors of kinases within this pathway. The present invention provides methods of using known bisindolyl maleimides previously disclosed as selective inhibitors of protein kinase C beta-1 and protein kinase C beta-2 (see inter alia WO 01/30331). Specifically, inhibition of PDK-1 by these compounds would be expected to suppress activation of the entire pathway as PDK- 1 is the key kinase activating AKT. Inhibition of p70S6 kinase, a kinase effector downstream of AKT, would further suppress the enhanced ribosome biogenesis and protein translation triggered by AKT pathway activation.

Prostatic adenocarcinoma (CaP) is the most common, non-cutaneous malignancy and the second-leading cause of cancer death in men. The disease has two distinct phases: the androgen-dependent phase, which can be treated effectively with androgen ablation therapies, and the androgen-independent phase. It is estimated that over thirty thousand men will die each year from androgen-independent metastatic CaP. Efforts to understand the metastatic progression of CaP progression to androgen-independent, metastatic disease involves a dampened apoptotic response, a release from the cell cycle block that initially follows androgen withdrawal and a shift from dependence on paracrine-derived growth and survival factors to autonomous production of these key proteins. Functional loss of the tumor suppressor phosphatase and tensin homologue deleted on the chromosome ten (PTEN) and subsequent activation of the AKT pathway, have been prominently implicated in the progression of CaP to androgen-independence.

Activation of the AKT pathway can suppress the apoptotic response, undermine cell cycle control and selectively enhance the production of key growth and survival factors. Though many proteins and intracellular signaling pathways can influence these biological responses, activation of the AKT pathway is a particularly potent signal involved in CaP progression to androgen-independence and therefore provides a therapy of advanced androgen-independent CaP (Graff 2002). Treatment of CWR22Rv1, LNCaP and Du145 prostate cancer cells with the compound induces apoptosis.

Rokhlin et al. J. Biol. Chemistry 2002, 277, 36, 33213-33219 reports that a specific bisindolylmaleimide compound Bis IX was able to inhibit transcription in TNF receptor family-mediated cell death. Bis IX is reportedly able to potentiate cell death when used in combination with an apoptosis inducing agent such as TNF-α, agonistic anti-Fas monoclonal antibody and TNF-related apoptosis-inducing ligand (TRAIL) by inducing caspase activity. Bis IX reportedly cannot induce caspase activity alone. Rokhlin et al. concluded that such combination therapy with apoptosis inducing agents such as TRAIL may be useful in treating androgen-independent prostate cancer.

### SUMMARY OF THE INVENTION

The present invention provides use of a compound of the formula 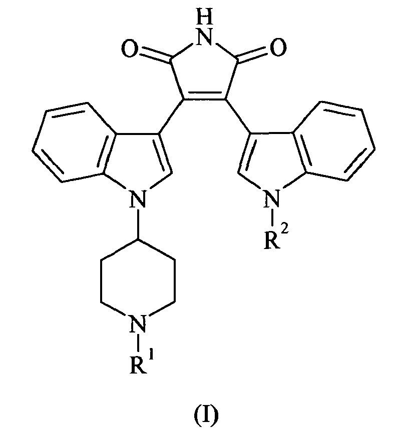 wherein R¹ and R² are each independently hydrogen or C₁-C₄ alkyl; or a pharmaceutically acceptable salt thereof, in the preparation of a medicament for treating prostate cancer.

In a second embodiment, the invention provides use of a compound of formula I or a pharmaceutically acceptable salt thereof, in the preparation of a medicament for treating androgen-independent prostatic adenocarcinoma.

In a third embodiment, the invention provides use of a compound of formula I or a pharmaceutically acceptable salt thereof in the preparation of a medicament for treating an glioblastoma, colon cancer, pancreatic cancer, ovarian cancer, endometrial cancer, or renal cell cancer.

### DETAILED DESCRIPTION OF THE INVENTION

General terms used in the description of compounds herein described bear their usual meanings. For example, the term "C₁-C₄ alkyl" refers to straight or branched, monovalent, saturated aliphatic chains of 1 to 4 carbon atoms and includes, but is not limited to, methyl, ethyl, propyl, isopropyl, butyl, isobutyl, sec-butyl, and *tert*-butyl.

Preferred compounds of this invention include compounds of formula I wherein R¹ is hydrogen, methyl, ethyl, n-propyl, or isopropyl. Further preferred compounds include those wherein R² is hydrogen or methyl. More preferred compounds are those where R¹ is hydrogen. The skilled artisan will appreciate that additional preferred embodiments may be selected by combining the preferred embodiments above, or by reference to the examples given herein.

The term "pharmaceutically-acceptable salt" as used herein, refers to a salt of a compound of the above Formula (I). It should be recognized that the particular counterion forming a part of any salt of this invention is usually not of a critical nature, so long as the salt as a whole is pharmacologically acceptable and as long as the counterion does not contribute undesired qualities to the salt as a whole.

The compounds of Formula (I) described herein form pharmaceutically-acceptable acid addition salts with a wide variety of organic and inorganic acids and include the physiologically-acceptable salts which are often used in pharmaceutical chemistry. Such salts are also part of this invention. A pharmaceutically-acceptable acid addition salt is formed from a pharmaceutically-acceptable acid, as is well known in the art. Such salts include the pharmaceutically acceptable salts listed in Journal of Pharmaceutical Science, 66, 2-19 (1977), which are known to the skilled artisan. *See also,* The Handbook of Pharmaceutical Salts; Properties, Selection, and Use. P. H. Stahl and C. G. Wermuth (ED.s), Verlag, Zurich (Switzerland) 2002.

Typical inorganic acids used to form such salts include hydrochloric, hydrobromic, hydriodic, nitric, sulfuric, phosphoric, hypophosphoric, metaphosphoric, pyrophosphoric, and the like. Salts derived from organic acids, such as aliphatic mono and dicarboxylic acids, phenyl substituted alkanoic acids, hydroxyalkanoic and hydroxyalkandioic acids, aromatic acids, aliphatic and aromatic sulfonic acids, may also be used. Such pharmaceutically acceptable salts thus include acetate, phenylacetate, trifluoroacetate, acrylate, ascorbate, benzoate, chlorobenzoate, dinitrobenzoate, hydroxybenzoate, methoxybenzoate, methylbenzoate, o-acetoxybenzoate, naphthalene-2-benzoate, bromide, isobutyrate, phenylbutyrate, α-hydroxybutyrate, butyne-1,4-dicarboxylate, hexyne-1,4-dicarboxylate, caprate, caprylate, cinnamate, citrate, formate, fumarate, glycollate, heptanoate, hippurate, lactate, malate, maleate, hydroxymaleate, malonate, mandelate, mesylate, nicotinate, isonicotinate, nitrate, oxalate, phthalate, teraphthalate, propiolate, propionate, phenylpropionate, salicylate, sebacate, succinate, suberate, benzenesulfonate, p-bromobenzenesulfonate, chlorobenzenesulfonate, ethylsulfonate, 2-hydroxyethylsulfonate, methylsulfonate, naphthalene-1-sulfonate, naphthalene-2-sulfonate, naphthalene-1,5-sulfonate, p-toluenesulfonate, xylenesulfonate, tartarate, and the like.

The compounds of formula (I) are described in Heath, Jr. et al., U.S. Pat. No. 5,668,152. The synthesis of the compounds of formula (I) are fully set forth as well as a disclosure that said compounds are useful as beta-1 and beta-2 isozyme selective protein kinase C (PKC) inhibitors. As isozyme selective PKC inhibitors, the compounds have previously been disclosed as useful in the treatment of conditions associated with diabetes mellitus and its complications as well ischemia, inflammation, central nervous system disorders, cardiovascular disease, dermatological disease, Alzheimer's disease and cancer.

While U.S. Pat. No. 5,668,152 describes the treatment of cancer using PKC beta-1 and beta-2 selective inhibitors, of which the present compounds of formula (I) are included generically, there is no teaching or suggestion that the compounds of formula (I) are inhibitors of the PI3K/ AKT pathway. Because the AKT pathway acts as a central regulator of the apoptotic response, inhibitors of this pathway would be expected to induce apoptosis and/ or block cell cycle progression whereas inhibition of PKC, which has many disparate roles in the cell, would not necessarily be expected to do so.

As used herein, the term "patient" refers to a warm-blooded animal or mammal which is in need of treating, or at risk of developing, one or more diseases or disorders associated with AKT pathway activity (e.g. PDK-1/ p70S6 kinase activity). It is understood that guinea pigs, dogs, cats, rats, mice, hamsters, and primates, including humans, are examples of patients within the scope of the meaning of the term. Preferred patients include humans.

The compounds of the present invention can be administered alone or in the form of a pharmaceutical composition, that is, combined with pharmaceutically acceptable carriers, or excipients, the proportion and nature of which are determined by the solubility and chemical properties of the compound selected, the chosen route of administration, and standard pharmaceutical practice. The compounds of the present invention, while effective themselves, may be formulated and administered in the form of their pharmaceutically acceptable salts, for purposes of stability, convenience of crystallization, increased solubility, and the like.

Thus, the present invention provides pharmaceutical compositions comprising a compound of the Formula (I) and a pharmaceutically acceptable diluent.

The compounds of Formula (I) can be administered by a variety of routes. In effecting treatment of a patient afflicted with or at risk of developing the disorders described herein, a compound of Formula (I) can be administered in any form or mode that makes the compound bioavailable in an effective amount, including oral and parenteral routes. For example, compounds of Formula (I) can be administered orally, by inhalation, or by the subcutaneous, intramuscular, intravenous, transdermal, intranasal, rectal, occular, topical, sublingual, buccal, or other routes. Oral administration is generally preferred for treatment of the disorders described herein. However, oral administration is not the only preferred route. For example, the intravenous route may be preferred as a matter of convenience or to avoid potential complications related to oral administration. When the compound of Formula (I) is administered through the intravenous route, an intravenous bolus or slow infusion is preferred.

One skilled in the art of preparing formulations can readily select the proper form and mode of administration depending upon the particular characteristics of the compound selected, the disorder or condition to be treated, the stage of the disorder or condition, and other relevant circumstances. (*Remington's Pharmaceutical Sciences,* 18th Edition, Mack Publishing Co. (1990)).

The pharmaceutical compositions are prepared in a manner well known in the pharmaceutical art. The carrier or excipient may be a solid, semi-solid, or liquid material that can serve as a vehicle or medium for the active ingredient. Suitable carriers or excipients are well known in the art. The pharmaceutical composition may be adapted for oral, inhalation, parenteral, or topical use and may be administered to the patient in the form of tablets, capsules, aerosols, inhalants, suppositories, solutions, suspensions, or the like.

For the purpose of oral therapeutic administration, the compounds may be incorporated with excipients and used in the form of tablets, troches, capsules, elixirs, suspensions, syrups, wafers, chewing gums and the like. These preparations should contain at least 4% of the compound of the present invention, the active ingredient, but may be varied depending upon the particular form and may conveniently be between 4% to about 70% of the weight of the unit. The amount of the compound present in compositions is such that a suitable dosage will be obtained. Preferred compositions and preparations according to the present invention may be determined by a person skilled in the art.

The tablets, pills, capsules, troches, and the like may also contain one or more of the following adjuvants: binders such as povidone, hydroxypropyl cellulose, microcrystalline cellulose, gum tragacanth or gelatin; excipients such as dicalcium phosphate, starch, or lactose; disintegrating agents such as alginic acid, Primogel, corn starch and the like; lubricants such as talc, hydrogenated vegetable oil, magnesium stearate or Sterotex; glidants such as colloidal silicon dioxide; and sweetening agents, such as sucrose, aspartame, or saccharin, or a flavoring agent, such as peppermint, methyl salicylate or orange flavoring, may be added. When the dosage unit form is a capsule, it may contain, in addition to materials of the above type, a liquid carrier such as polyethylene glycol or a fatty oil. Other dosage unit forms may contain other various materials that modify the physical form of the dosage unit, for example, coatings. Thus, tablets or pills may be coated with sugar, shellac, or other coating agents. Syrups may contain, in addition to the.present compounds, sucrose as a sweetening agent and certain preservatives, dyes and colorings and flavors. Materials used in preparing these various compositions should be pharmaceutically pure and non-toxic in the amounts used.

The compounds of Formula (I) are inhibitors of PDK1 and p70S6 kinase, two members of the PI3kinase/ AKT pathway. The inhibitory activity of the compounds of Formula (I) may be demonstrated by the methods below.

### Kinase Activity Assays

The assay described measures the phosphorylation of the PDK1 consensus phosphorylation site PDK-tide peptide (KTFCGTPEYLAPEVRREPRILSEEEQEMFRDFDYIADWC; cat # 14-452, lot 23876U) by recombinant PDK-1 (UBI) at Km for ATP and PDKtide saturation using phosphocellulose membrane filter plates. Phosphorylation of the p70S6 kinase substrate by recombinant p70S6 kinase is also measured similarly.

### Cell culture, Drug Treatment, Apoptosis and Proliferation Assays

Both HCT116 colon carcinoma (cat#CCL-247) and U87MG glioblastoma (cat#HTB-14) cell lines were obtained from the American Type Culture Collection (ATCC). The standard growth media differed for each cell line but all were grown in 10% heat-inactivated FBS (Invitrogen cat# 10082-147), 37°C, 5% CO2 atmosphere and in a humidified chamber. Cell passage was completed one to two times per week using 0.25% trypsin / 1mM EDTA (Invitrogen,cat# 25200-056) solution maintaining cells in log phase growth. U87MG cells were cultured in DMEM media (Invitrogen cat# 11965-092), 1mM non-essential amino acids (NEAA), and 0.1mM Sodium Pyruvate. HCT116 cells were grown in McCoy's 5A Modified media (Invitrogen, cat# 16600-082), 0.15% sodium bicarbonate, 0.1mM HEPES, 25mM D-glucose and 0.1mM sodium Pyruvate.

Apoptosis assays were executed using the Cell Death Detection ELISA^{plus} (Roche, 1774425) assay kit strictly following the enclosed protocol.

Changes in cellular proliferation resulting from treatment with LY317615 (see for example WO 02/02116), which is a compound of the formula 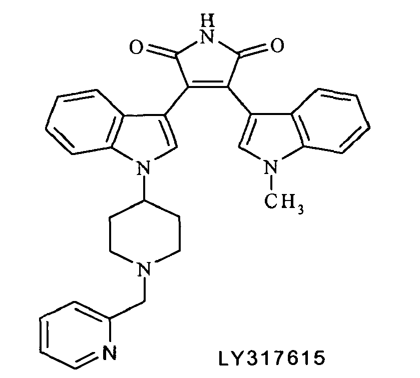 or a compound of formula (I) were R¹ is hydrogen and R² is methyl (Compound 1) were assessed by incorporation of propidium iodide (PI) (Sigma, cat# p-4864). Briefly, each cell culture plate was centrifuged 10 minutes (200rpm), the supernatant was gently aspirated and 100 µl 0.125 mM PI in PBS was added to each well of a 96-well plate. The fluorescence intensity of each well in the culture was measured (non-viable cells) using the Vector² multi-channel plate reader (Wallac, model#1420) and frozen to -80° C. The plate was allowed to thaw, come to room temperature and re-analyzed for changes in fluorescence intensity (total cells) again using the Vector². The proliferating cells in the culture were determined by subtracting the non-viable fraction from the total cells. The results were then reported as a percent of the un-treated control.

Protein lysates were prepared by incubation in RIPA Buffer (50 mM Tris -HCl, 150 mM NaCl, 1 mM EDTA , 1% NP-40, 0.25% Sodium deoxycholate, 1 mM sodium fluoride, 1mM sodium orthovanadate and Complete™ protease inhibitors (Roche Cat# 10019600) for one hour with rotation. The lysate was then centrifuged (10 minutes@ 10,000 rpm), supernatant harvested and protein concentrations were determined using the Bio-Rad DC Protein Assay (cat# 500-0122). Proteins were separated by SDS-PAGE using 4-20% tris-glycine gels (Invitrogen, cat# EC6028) and transferred to Hyper™-bond PVDF membrane (Amersham, cart# rpn303F). All primary antibodies were incubated overnight at 4° C in 5% milk / 1X PBS (Gibco, cat# 70011-044) solution. Horseradish peroxidase (HRP) linked secondary antibodies (Santa Cruz, cat# sc-2055, sc-2054) were incubated for a minimum of two hours prior to detection. Specific signal was determined by the Lumi-Imager™ and Lumi-Analyst software to define changes in protein expression and phosphorylation. The primary antibodies used are as follows: GSK3b, pGSK3b^{ser9} (Cell Signaling, cat#9332, 9336), S6 ribosomal protein, pS6 ribosomal protein^{ser240/244} (Cell Signaling, cat#2212, 2215), AKT (Transduction Labs cat#610861), pAKT^{ser308}, pAKT^{ser473} (Cell Signaling, cat#9275, 9271), PHASi (Zymed, cat#51-2900), p4EBP1^{ser65} (Cell Signaling cat#9451), p70S6 kinase, phos-p70S6 kinase^{thr421/ser424} (Cell Signaling cat#9202, 9204), p90RSK, phos-p90RSK^{thr359/ser363} (Cell Signaling cat#9347, 9344), FKHRL1, pFKHRL^{thr32} (Upstate Biochemicals, Inc. cat#06-951, 06952).

### Experimental Protocol for In. Vivo Tumor Inhibition Studies

Approximately 5 x10⁶ tumor cells are implanted subcutaneously into the flank of athymic nude mice (Harlan, Indianapolis, IN). Treatment of tumors begins when the tumors reach 100mm³ and continues for 21 consecutive days twice per day PO. Body weight and tumor size are monitored weekly or twice weekly.

### Results

Compound 1 inhibits PDK-1 with an EC50 of 370 nM and inhibits p70S6kinase with an EC50 <500nM. Treatment with Compound 1 induces apoptosis in human cancer cell lines derived from colon, lung, and prostate (both androgen-dependent and independent cell lines) as well as from non-Hodgkin's lymphoma. Treatment with Compound 1 suppresses phosphorylation of GSK3β, the forkhead transcription factor AFX, 4EBP1, and ribosomal protein S6- all readouts of AKT pathway activity. Furthermore, treatment of human tumor xenograft-bearing mice with Compound 1 suppresses GSK3β ser9 phosphorylation in these xenograft tissues, including an androgen-independent prostate carcinoma cell line, for up to 8 hours post dosing. Antitumor efficacy of the compound has been demonstrated in both HCT116 colon cancer xenografts, in U87MG glioblastoma xenografts and in xenografts from the androgen-independent prostate carcinoma cell line PC3.

## Claims

1. Use of a compound of the formula 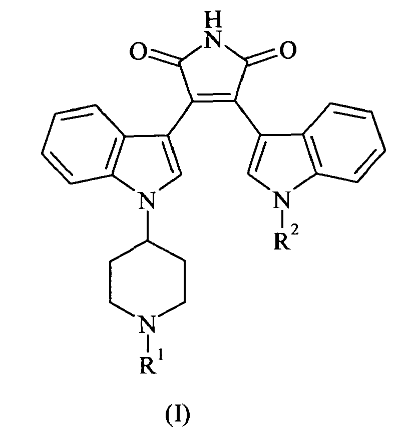 wherein R¹ and R² are each independently hydrogen or C₁-C₄ alkyl; or a pharmaceutically acceptable salt thereof, in the preparation of a medicament for treating prostate cancer.

2. Use of a compound of the formula 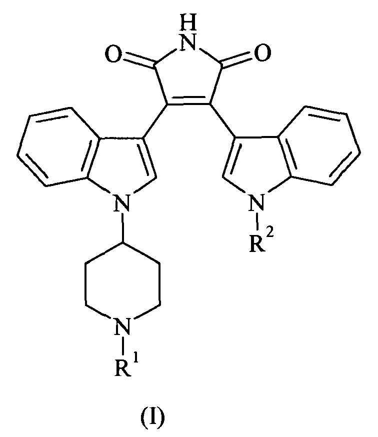 wherein R¹ and R² are each independently hydrogen or C₁-C₄ alkyl; or a pharmaceutically acceptable salt thereof, in the preparation of a medicament for treating androgen-independent prostatic adenocarcinoma.

3. Use of a compound of the formula 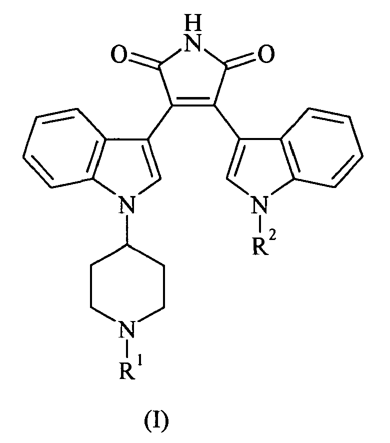 wherein R¹ and R² are each independently hydrogen or C₁-C₄ alkyl; or a pharmaceutically acceptable salt thereof in the preparation of a medicament for treating glioblastoma, colon cancer, pancreatic cancer, ovarian cancer, endometrial cancer, or renal cell cancer.

4. Use according to claim 3 wherein said medicament is for treating glioblastoma.

5. Use according to claim 3 wherein said medicament is for treating colon cancer.

6. Use according to claim 3 wherein said medicament is for treating pancreatic cancer.

7. Use according to claim 3 wherein said medicament is for treating ovarian cancer.

8. Use according to claim 3 wherein medicament is for treating endometrial cancer.

9. Use according to claim 3 wherein said medicament is for treating renal cell cancer.

10. Use according to claim 1 or claim 2 wherein R² is hydrogen or methyl.

11. Use according to claim 1 or claim 2 wherein R¹ is hydrogen, methyl, ethyl, n-propyl, or isopropyl.

12. Use according to any one of claims 1 to 9 wherein R¹ is hydrogen and R² is methyl.

## Patentansprüche

1. Verwendung einer Verbindung der Formel 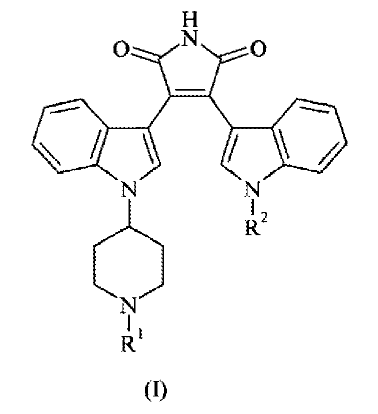 worin R¹ und R² jeweils unabhängig für Wasserstoff oder C₁-C₄-Alkyl stehen, oder eines pharmazeutisch akzeptablen Salzes hiervon, zur Herstellung eines Arzneimittels für die Behandlung von Prostatakrebs.

2. Verwendung einer Verbindung der Formel 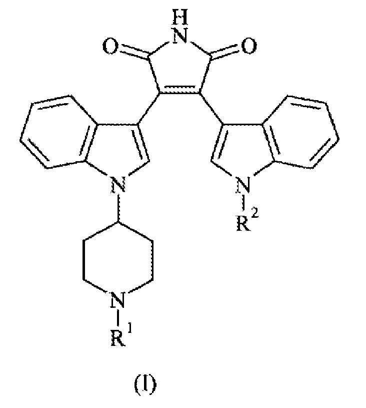 worin R¹ und R² jeweils unabhängig für Wasserstoff oder C₁-C₄-Alkyl stehen, oder eines pharmazeutisch akzeptablen Salzes hiervon, zur Herstellung eines Arzneimittels für die Behandlung von Androgen-unabhängigem Prostataadenokarzinom.

3. Verwendung einer Verbindung der Formel 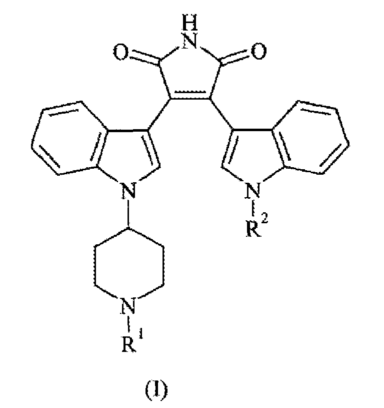 worin R¹ und R² jeweils unabhängig für Wasserstoff oder C₁-C₄-Alkyl stehen, oder eines pharmazeutisch akzeptablen Salzes hiervon, zur Herstellung eines Arzneimittels für die Behandlung von Glioblastom, Kolonkrebs, Pankreaskrebs, Ovarialkrebs, Endometrialkrebs oder Renalzellenkrebs.

4. Verwendung nach Anspruch 3, worin das Arzneimittel für die Behandlung von Glioblastom ist.

5. Verwendung nach Anspruch 3, worin das Arzneimittel für die Behandlung von Kolonkrebs ist.

6. Verwendung nach Anspruch 3, worin das Arzneimittel für die Behandlung von Pankreaskrebs ist.

7. Verwendung nach Anspruch 3, worin das Arzneimittel für die Behandlung von Ovarialkrebs ist.

8. Verwendung nach Anspruch 3, worin das Arzneimittel für die Behandlung von Endometrialkrebs ist.

9. Verwendung nach Anspruch 3, worin das Arzneimittel für die Behandlung von Renalzellenkrebs ist.

10. Verwendung nach Anspruch 1 oder 2, worin R² für Wasserstoff oder Methyl steht.

11. Verwendung nach Anspruch 1 oder 2, worin R¹ für Wasserstoff, Methyl, Ethyl, n-Propyl oder Isopropyl steht.

12. Verwendung nach einem der Ansprüche 1 bis 9, worin R¹ für Wasserstoff steht und R² für Methyl steht.

## Revendications

1. Utilisation d'un composé de formule 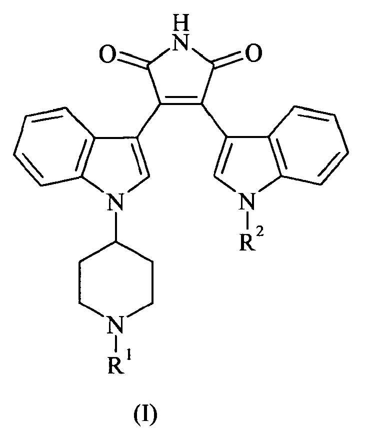 où R¹ et R² représentent, chacun indépendamment, hydrogène ou C₁-C₄-alkyle ; ou d'un sel pharmaceutiquement acceptable de celui-ci, dans la préparation d'un médicament destiné au traitement du cancer de la prostate.

2. Utilisation d'un composé de formule 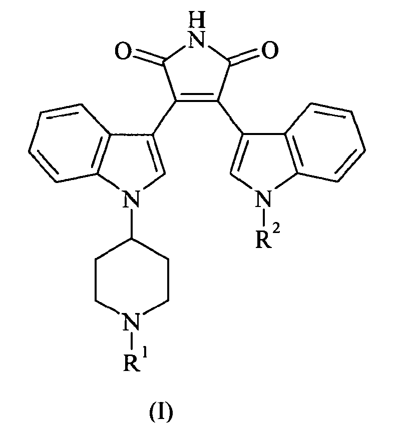 où R¹ et R² représentent, chacun indépendamment, hydrogène ou C₁-C₄-alkyle; ou d'un sel pharmaceutiquement acceptable de celui-ci, dans la préparation d'un médicament destiné au traitement de l'adénocarcinome prostatique indépendant des androgènes.

3. Utilisation d'un composé de formule 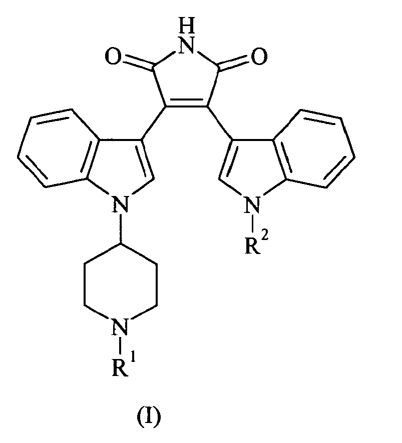 où R¹ et R² représentent, chacun indépendamment, hydrogène ou C₁-C₄-alkyle ; ou d'un sel pharmaceutiquement acceptable de celui-ci, dans la préparation d'un médicament destiné au traitement du glioblastome, du cancer du côlon, du cancer pancréatique, du cancer ovarien, du cancer de l'endomètre ou du cancer des cellules rénales.

4. Utilisation selon la revendication 3, où ledit médicament est destiné au traitement du glioblastome.

5. Utilisation selon la revendication 3, où ledit médicament est destiné au traitement du cancer du côlon.

6. Utilisation selon la revendication 3, où ledit médicament est destiné au traitement du cancer pancréatique.

7. Utilisation selon la revendication 3, où ledit médicament est destiné au traitement du cancer ovarien.

8. Utilisation selon la revendication 3, où ledit médicament est destiné au traitement du cancer de l'endomètre.

9. Utilisation selon la revendication 3, où ledit médicament est destiné au traitement du cancer des cellules rénales.

10. Utilisation selon la revendication 1 ou 2 où R² représente hydrogène ou méthyle.

11. Utilisation selon la revendication 1 ou 2 où R¹ représente hydrogène, méthyle, éthyle, n-propyle ou isopropyle.

12. Utilisation selon l'une quelconque des revendications 1 à 9 où R¹ représente hydrogène et R² représente méthyle.
